# EUROPEAN PATENT APPLICATION

(11) **EP 2 636 379 A1**
(43) Date of publication of application: **11.09.2013**
(21) Application number: 13158091.2
(22) Date of filing: 07.03.2013
(51) Int. Cl.: A61B 17/29, A61B 10/06, A61B 10/02, A61B 17/00

(54) **Tissue dissection and removal device**

(30) Priority: 08.03.2012 US 201261608241 P; 25.02.2013 US 201313775292
(71) Applicant: Covidien LP, Mansfield, MA 02048 (US)
(72) Inventor: Rethy, Csaba L., Westport, CT Connecticut 06880 (US); Krehel, Gregg, Newtown, CT Connecticut 06470 (US)
(74) Representative: Soames, Candida Jane

(57) **Abstract**

A surgical instrument includes a handle portion and a body portion extending distally from the handle portion and defining a longitudinal axis. The surgical instrument also includes an end effector assembly disposed at a distal end of the body portion. The end effector assembly having an elongate member has a cavity for the reception of a tissue sample therein and a closing member for securely enclosing the tissue sample within the cavity.

## Description

### CROSS REFERENCE TO RELATED APPLICATION

The present application claims the benefit of and priority to U.S. Provisional Application Serial No. 61/608,241, filed on March 8, 2012, the entire contents of which are incorporated herein by reference.

### BACKGROUND

### Technical Field

The present disclosure is generally directed to a system and method for capturing body tissue samples. The present disclosure is more particularly directed to such a system and method which finds particular application for the isolation, resection, and capture of lymph nodes or other tissue.

### Background of Related Art

Many thoracic surgical procedures are performed for heart and lung disease, muscle and nerve disorders, ulcers and other serious illnesses. Although surgery may be the best, or only way to treat the disease, patients may sometimes face a long and difficult recovery because traditional "open" thoracic surgery is highly invasive. In an open thoracic surgery, known as a thoracotomy, surgeons must make a long incision through chest muscles and then cut and spread the patient's ribs to reach the diseased area. As a result, patients may spend up to a week in the hospital and up to four to six weeks of recovery at home.

The development of endoscopic video capability and instrumentation has resulted in the application of diagnostic and therapeutic thoracoscopy, also known as video-assisted thoracic surgery (VATS), to many disease processes encountered in thoracic medicine. VATS is a technique in which small diameter instruments such as cameras, graspers, forceps, retractors, dissectors, clamps, and so forth are inserted through small openings in the body to perform surgical procedures within the thoracic cavity. By utilizing a VATS procedure for exploring, diagnosing, and treating disease processes within the thoracic cavity, the pain, morbidity, and long recovery duration of more invasive procedures, such as the traditional large incision thoracotomy can often be avoided.

VATS procedures typically require double-lumen endotracheal intubation and single-lung ventilation. Working space in which to maneuver surgically in the chest is created by ventilating the opposite lung through the double-lumen endotracheal tube and allowing the collapse of the affected lung after creation of a small intercostal incision. Collapse of the affected lung enables improved visibility of the lung, as well as virtually all the major structures in the chest cavity, to aid in exploration, treatment, and/or biopsy of a target site. However, double-lumen endotracheal intubation and single-lung ventilation techniques still present limitations when extracting tissue samples is involved.

Therefore, there is a need in the art for an improved system and method for the capturing of body tissue samples. More particularly, there is a need for a system and method for capturing bronchial and thoracic lymph nodes suspected of being cancerous.

### SUMMARY

In one aspect of the present disclosure, a surgical instrument is provided including a handle portion and a body portion extending distally from the handle portion and defining a longitudinal axis. The surgical instrument also includes an end effector assembly disposed at a distal end of the body portion. The end effector assembly includes a distal tip configured for dissection of tissue, an elongate member having a cavity for the reception of a tissue sample therein and a closing member for securely enclosing the tissue sample within the cavity.

In one exemplary embodiment, the end effector assembly is rotatable about the longitudinal axis. The body portion can include an articulation mechanism for articulating the end effector assembly.

A portion of the closing member can include a blade member.

In yet another exemplary embodiment, the closing member is pivotally coupled to the elongate member.

In some embodiments, the elongate member includes a plurality of cavities, each cavity dimensioned and adapted to receive a different tissue sample, and each cavity including a respective closing member, wherein access to each cavity is controlled by an actuating unit. The elongate member in some embodiments includes at least two cavities disposed on opposed sides thereof.

In some embodiments, the end effector assembly is removably attachable to the body portion.

In accordance with another aspect of the present disclosure, an end effector assembly is provided including an elongate member including a cavity for the reception of a tissue sample therein and a closing member for securely enclosing the tissue sample within the cavity.

In another aspect of the present disclosure, a method of removing tissue from a thoracic cavity is provided. The method includes the steps of providing a surgical instrument including a handle portion, a body portion extending distally from the handle portion and defining a longitudinal axis and having a dimension for insertion through an access port, and an end effector assembly disposed at a distal end of the body portion. The end effector assembly has an elongate member having a cavity for the reception of a tissue sample therein and a closing member for securely enclosing the tissue sample within the cavity. Further steps include introducing the surgical instrument into the thoracic cavity of a patient, dissecting the target tissue via a distal end of the elongate member, advancing the end effector assembly toward a target tissue within the thoracic cavity, opening the closing member to receive the target tissue within the cavity of the elongated member, closing the closing member to sever a tissue sample from the target tissue and to seal the tissue sample within the cavity and removing the end effector assembly from the patient with the tissue sample secured within the cavity.

In some embodiments, the method further includes the step of articulating the end effector assembly with respect to a longitudinal axis of the body portion.

In some embodiments, the end effector is removably coupled to the body portion.

In some embodiments, the instrument includes a second cavity formed on the elongate member, each cavity dimensioned and adapted to receive a different tissue sample, and the second cavity includes a second closing member. In some embodiments, the first and second cavities are disposed on opposed sides thereof; in other embodiments, the first and second cavities are disposed in substantial longitudinal alignment. The method can include the step of closing a second closing member to sever a second tissue sample from surrounding tissue and seal the severed second tissue sample in the second cavity. Each cavity can be dimensioned and adapted to receive different tissue samples, and each cavity can include a respective closing member, wherein access to each cavity is controlled by an actuating unit.

In some embodiments, the surgical instrument is introduced into the thoracic cavity during a video assisted thoracic surgery procedure.

In some embodiments, the surgical instrument retrieves lymph node for pathology.

Further scope of applicability of the present disclosure will become apparent from the detailed description given hereinafter. However, it should be understood that the detailed description and specific examples, while indicating preferred embodiments of the present disclosure, are given by way of illustration only, since various changes and modifications within the spirit and scope of the present disclosure will become apparent to those skilled in the art from this detailed description.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a perspective view of a surgical instrument, in accordance with one embodiment of the present disclosure;

FIG. 1A is a perspective view of another embodiment of the surgical instrument of the present disclosure;

FIG. 1B is a perspective view of another embodiment of the surgical instrument of the present disclosure;

FIGS. 2 and 2A are enlarged perspective views of an end effector assembly of the surgical instrument of FIG. 1, the end effector assembly including a cavity for receiving a tissue sample therein and a closing member for securing the tissue within the cavity;

FIG. 3A is an enlarged perspective view of an end effector assembly of an alternate embodiment including a plurality of cavities for receiving different tissue samples, each cavity including its own respective closing member;

FIGS. 3B-3E illustrate the first cavity of the end effector of FIG. 3A receiving a first tissue sample;

FIGS. 3F-3I illustrate the second cavity of the end effector of FIG. 3A receiving a second tissue sample;

FIG. 4A is an enlarged perspective view of an end effector assembly with opposed cavities positioned therein, in accordance with yet another embodiment of the present disclosure;

FIG. 4B illustrates the end effector of FIG. 4A rotated about 90 degrees from the position of FIG.4A to a second position to illustrate the opposed cavities positioned therein;

FIGS. 4C-4F illustrate the first cavity of the end effector of FIG. 4A receiving a first tissue sample; and

FIGS. 4G-4J illustrate the second cavity of the end effector of FIG. 4B receiving a second tissue sample.

The figures depict preferred embodiments of the present disclosure for purposes of illustration only. One skilled in the art will readily recognize from the following discussion that alternative embodiments of the structures and methods illustrated herein may be employed without departing from the principles of the present disclosure described herein.

### DETAILED DESCRIPTION

Embodiments of the presently disclosed apparatus will now be described in detail with reference to the drawings, in which like reference numerals designate identical or corresponding elements in each of the several views. As used herein, the term "distal" refers to that portion of the tool, or component thereof which is further from the user while the term "proximal" refers to that portion of the tool or component thereof which is closer to the user.

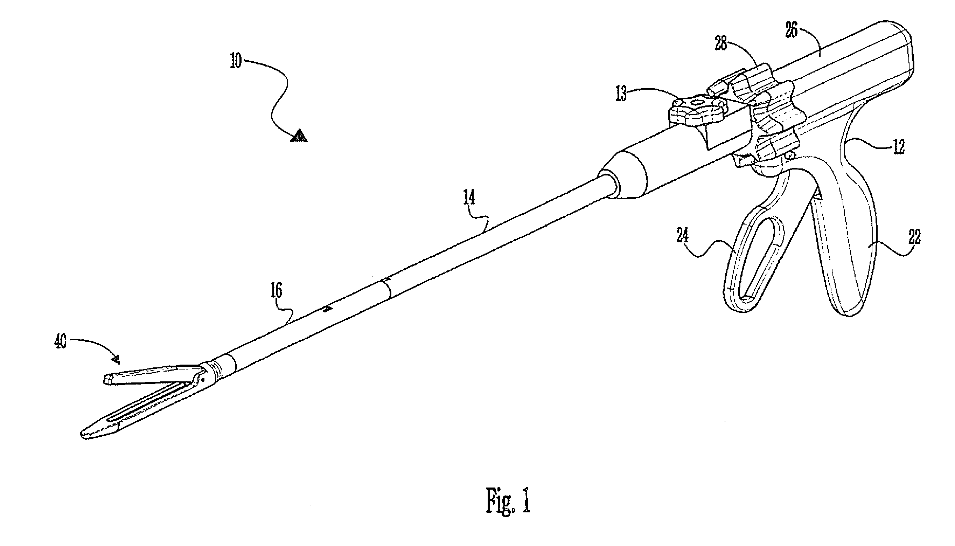

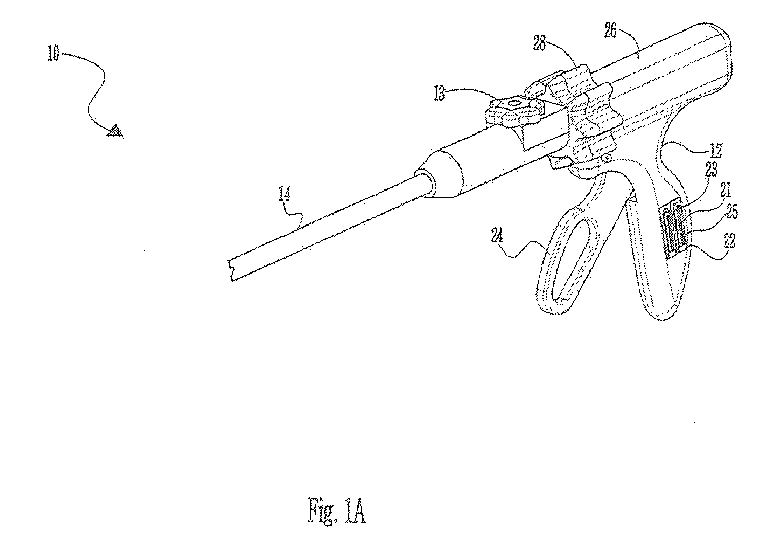

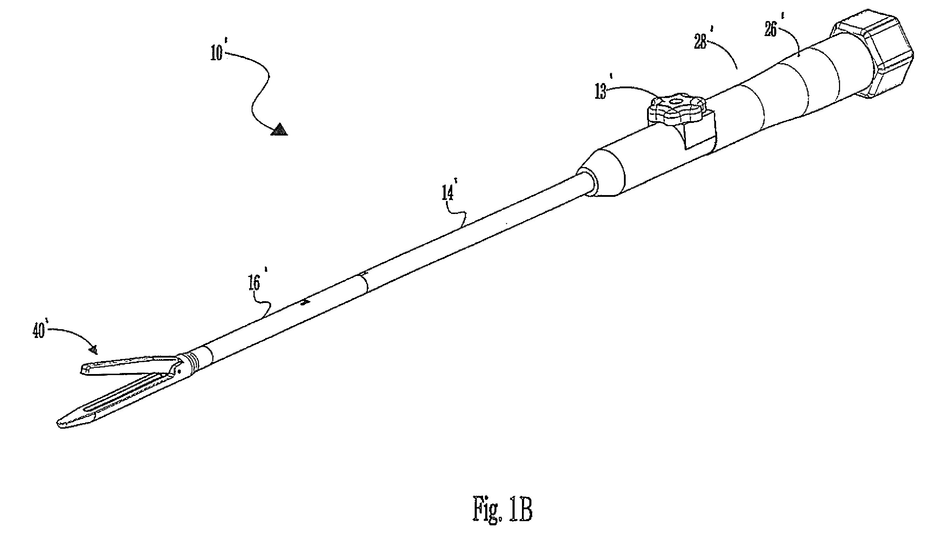

Referring to FIG. 1, a surgical instrument 10 in accordance with the present disclosure is illustrated. Surgical instrument 10 is an endoscopic apparatus and includes a handle assembly 12 and an elongate body 14 extending distally therefrom. The instrument can be 5mm in outer diameter, although other dimensions are also contemplated. A loading unit 16 which can be replaceable and can be disposable, can be releasably secured to the distal end of elongate body 14. The loading unit supports the end effector assembly 40.

Handle assembly 12 includes a stationary handle member 22, a movable handle member 24, and a barrel portion 26. A rotatable knob 28 is mounted on the barrel portion 26 to facilitate rotation of elongate body 14 with respect to handle assembly 12 about the longitudinal axis. An articulation knob 13 is mounted on the distal end of barrel portion 26, in proximity to rotatable knob 28, to facilitate articulation of tool assembly 40 to angular positions with respect to the longitudinal axis of elongated body 14.

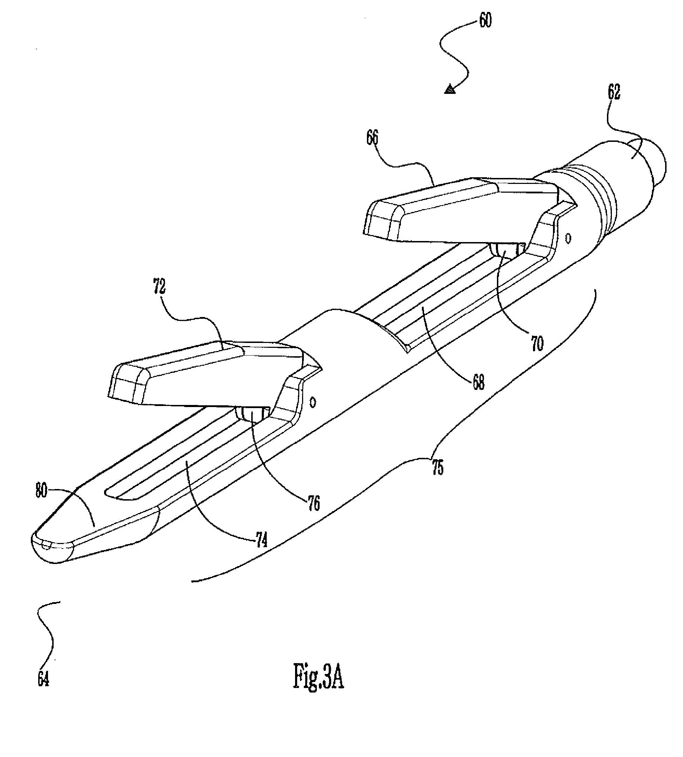

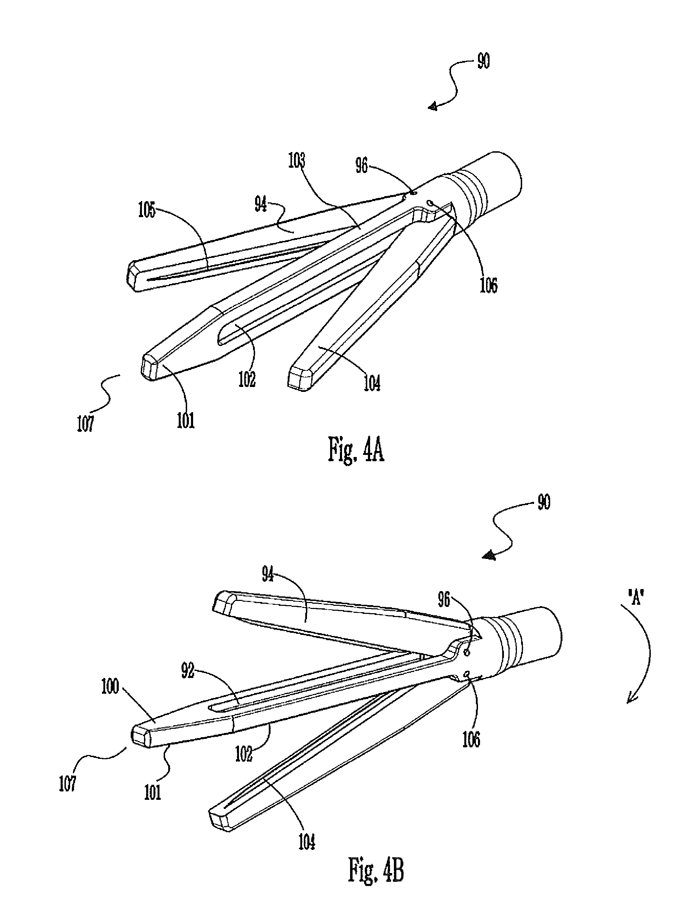

Additionally, the handle assembly 12 if used with the multiple jaw assemblies of Figures 3A-4J may also include an actuating unit 21 as shown in FIG. 1A. Actuation unit 21 may include a slidable mechanism 25 for switching between jaw assemblies, as described below with reference to FIGS. 3A-3I and 4A-4J. For example, a knob 23 may be located at a first position to actuate a jaw member of the one jaw assembly. When the user wishes to switch to a second jaw member, the user may slide the knob 23 via the sliding mechanism 25 to a second position to enable actuation of a jaw member of the other jaw assembly. Even though the actuating unit 21 is shown on the handle assembly 12, it is contemplated that the actuating unit 21 may be located on other portions of the surgical instrument 10. Also note that the end effector assemblies of Figures 1-4J can be supported on a loading unit, e.g. loading unit 16, and mounted to the elongate body 14 of the instrument 10. Alternately, the end effector assemblies can be fixedly (non-removably) mounted to the elongated body 14.

In the alternate embodiment of FIG. 1B, the handle assembly 28' of instrument 10' has an in line profile. Handle portion 26' is line with shaft or elongate body 14' and loading unit 16'. Knob 13' pivots jaw 40' between open and closed positions. Such in-line handle can be used with any of the jaw embodiments disclosed herein. The in-line handle may provide improved ergonomics in certain applications with the user grasping and maneuvering the shaft.

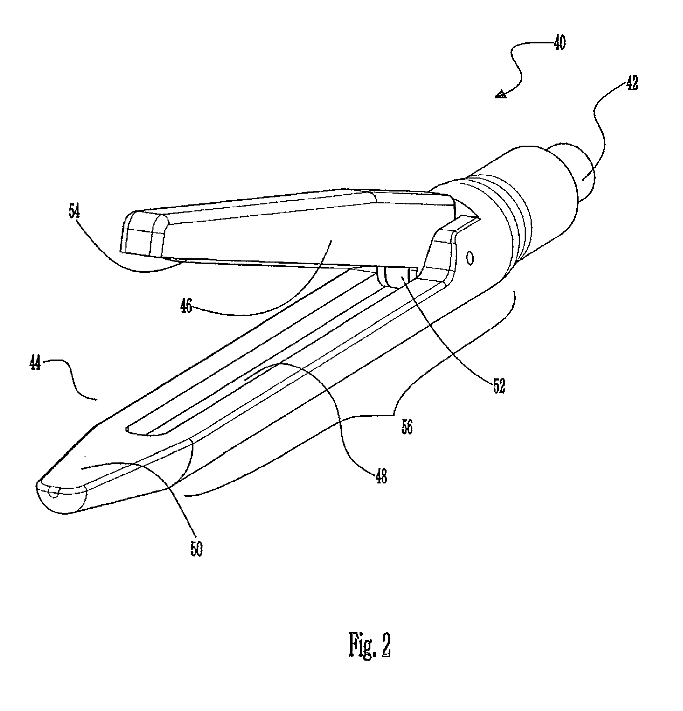

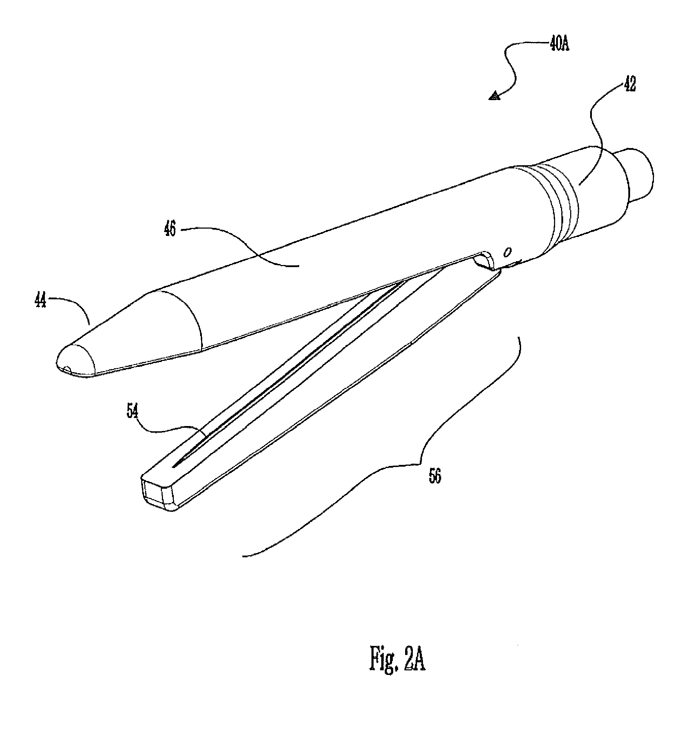

Referring to FIGS. 2 and 2A, the end effector assembly 40 includes a cavity 48 for receiving a tissue sample therein and a closing member 46 for securing the tissue within the cavity 48,

The end effector assembly 40 is attached to surgical instrument 10 at its proximal end 42 by a frictional fit, threaded engagement, or other methods. The end effector assembly 40 includes a tip 50 at its distal end 44. The tip 50 is shaped for blunt dissection of tissue. A cavity 48 is formed in an interior length of the end effector assembly 40. The cavity 48 is configured to receive a tissue sample therein. A closing member or jaw 46 is pivotally mounted via a pivot mechanism 52, e.g. a pivot pin, to the elongate member (or stationary jaw) 56 of the end effector assembly 40. The closing member 46 is configured to securely enclose within cavity 48 a tissue sample removed from tissue during a surgical procedure. The closing member 46 is adapted and dimensioned to be of a size approximately equal to the size of the opening or cavity 48 to fit therein to retain together with stationary jaw 56 the severed tissue sample.

The configuration of the distal tip 50 aids in the dissection of tissue as it navigates through the thoracic cavity. The tip 50 can be constructed in a sloping configuration. The tip 50 may have a conical or spherical outer face or edge. One skilled in the art may contemplate a plurality of different geometric configurations for the tip 50 for enabling the tip 50 to effectively dissect a portion of tissue.

The closing member 46 includes a blade member 54. The blade member 54 may be any type of cutting element, such as a chisel blade, that aids in the cutting of a tissue sample. The blade member 54 may extend the entire interior length of the closing member 46 and is preferably centrally disposed. However, it is contemplated that the blade member 54 could extend along only a portion of an interior length of the closing member 46. It is also contemplated that the blade could be off center. Additionally multiple blades could be provided.

The end effector assembly 40 may comprise any suitable shape and material. Solely by way of example, the end effector assembly 40 may comprise stainless steel or a biocompatible plastic. The end effector assembly 40 may be substantially cylindrically-shaped, when in the closed or clamped position. The end effector assembly 40 may be configured to articulate via the articulation knob 13 (see FIG. 1), as well as rotate about a longitudinal axis extending the length of the elongate body 14 of the surgical instrument 10.

In use, to perform a VATS procedure, the surgeon creates an incision, generally less than one inch in diameter, through an intercostal space (i.e., the area between the ribs) of a patient into a thoracic cavity. An access port may optionally be inserted into the incision to prevent the incision from closing. The access port could also be utilized in spread the tissue adjacent the opening. Endoscopic instrument 10, with closing member 46 in a closed position, is inserted through the port and into the thoracic cavity (not shown). The tip 50 can bluntly dissect tissue as the instrument 10 is advanced to the target tissue. After the end effector 40 of the endoscopic instrument 10 is in the desired location in the thoracic cavity, the surgeon may then adjust movable handle member 24 to deploy the end effector assembly 40 toward the target site as described below. The user may also rotate the end effector assembly 40 via rotation knob 28 and/or articulate end effector assembly 40 via rotation of knob 13 to adjust its position to facilitate access to the target tissue.

End effector assembly 40, extending from the distal end of elongate body 14, is then maneuvered within a working space to perform the desired diagnostic or surgical procedure, such as to remove a lymph node from tissue. Closing member 46 is moved to an open configuration by release of handle member 24 for receiving the lymph node. Once it is determined that the lymph node has been placed within the cavity 48, the handle member 24 is moved to cause the closing member 46 to move to a closed configuration. Such movement severs the tissue via blade 54 and secures the severed tissue within cavity 48. Once the lymph node has been securely sealed within the cavity 48, the end effector assembly 40 and instrument 10 are removed from the patient. The lymph node may be transferred to another storage vessel. The lymph node is delivered to a technician for further processing to determine, for example, whether the lymph node is cancerous.

Referring to FIG. 3A, an alternate embodiment of an end effector assembly in accordance with another embodiment is illustrated and designated generally by reference numeral 60. End effector assembly 60 includes two cavities 68, 74, spaced axially from another, for receiving different tissue samples, each cavity 68, 74 cooperating with a respective closing member or jaw 66, 72.

The end effector assembly 60 may be attached to surgical instrument 10 at its proximal end 62. The end effector assembly 60 includes a tip 80 at its distal end 64 which is configured like tip 50 of Figure 1 for blunt dissection. A first proximal cavity 68 and a second distal cavity 74 are disposed on a length of the end effector assembly 60. Each of the cavities 68, 74 is configured to receive a different tissue sample therein. A first closing member or movable jaw 66 is pivotally mounted via a pivot mechanism 70, e.g. a pivot pin, to the elongate member or stationary jaw 75 of the end effector assembly 60. Additionally, a second closing member or movable jaw 72 is pivotally mounted via a more distal pivot mechanism 76, e.g. a pivot pin, to the elongate member 75 of the end effector assembly 60. The closing members 66, 72 are configured to securely enclose different tissue samples removed from tissue during a surgical procedure. The closing members 66, 72 are adapted and dimensioned to be of a size approximately equal to the size of their respective cavities 68, 74 to encapsulate and seal a tissue sample disposed therein.

Each of the closing members includes a cutting blade as in the cutting blade (and its variations) discussed above.

The distal tip 80 aids in the dissection of tissue as it is advanced through the thoracic cavity. The tip 80 can be constructed in a sloping configuration. The tip 80 may have a conical or spherical outer face or edge. One skilled in the art may contemplate a plurality of different geometric configurations for the tip 80 for enabling the tip 80 to effectively dissect a portion of tissue.

One skilled in the art may contemplate incorporating three or more cavities along the length of the elongate member 75. The cavities may vary in size and shape. The cavities may also vary in shape and size with respect to each other on the same end effector assembly. Even though the cavities 68, 74 are shown to be in a series configuration, one skilled in the may contemplate a parallel configuration for cavities 68, 74. Moreover, one skilled in the art may contemplate a plurality of cavities in substantially equal spaced relation to each other or in non-equal spaced relation to each other.

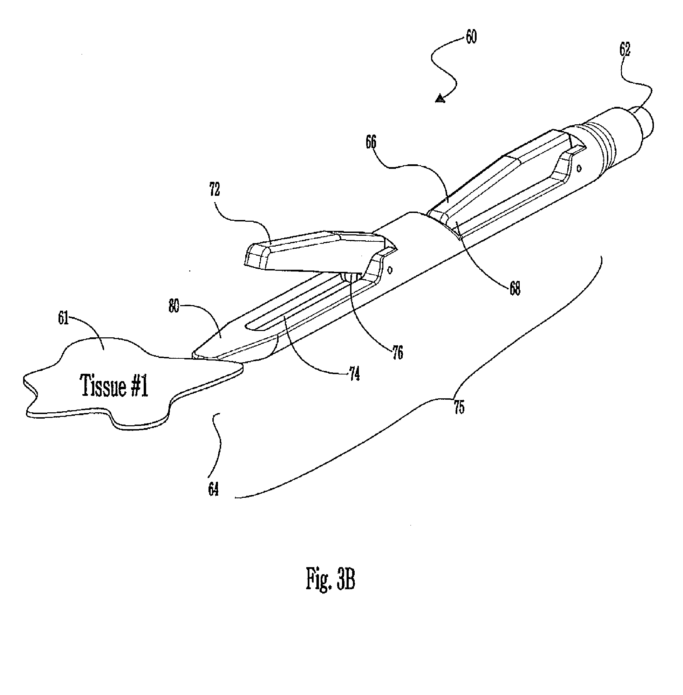

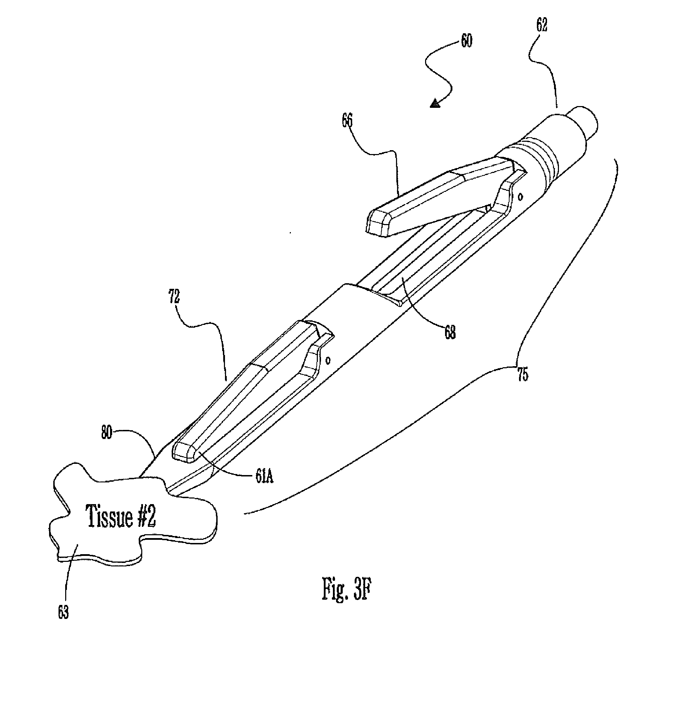

FIGS. 3B-3E illustrate the second (distal) cavity 74 of FIG. 3A receiving a first tissue sample 61, whereas FIGS. 3F-3I illustrate the first (proximal) cavity 68 of FIG. 3A receiving a second tissue sample 63, in accordance with an embodiment of the present disclosure.

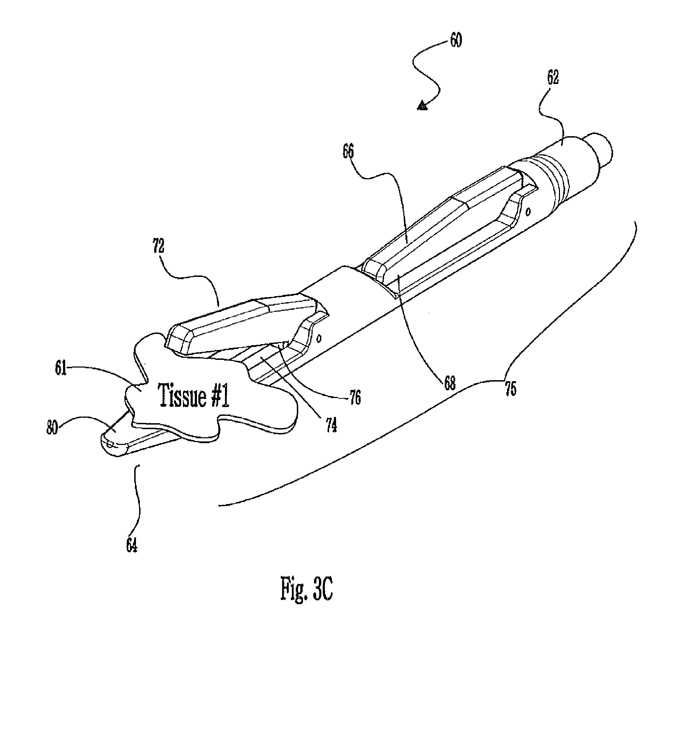

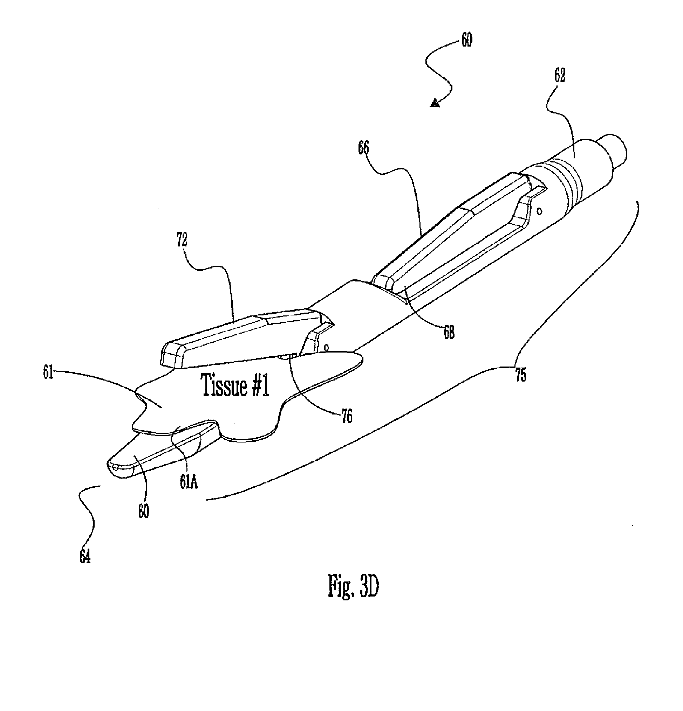

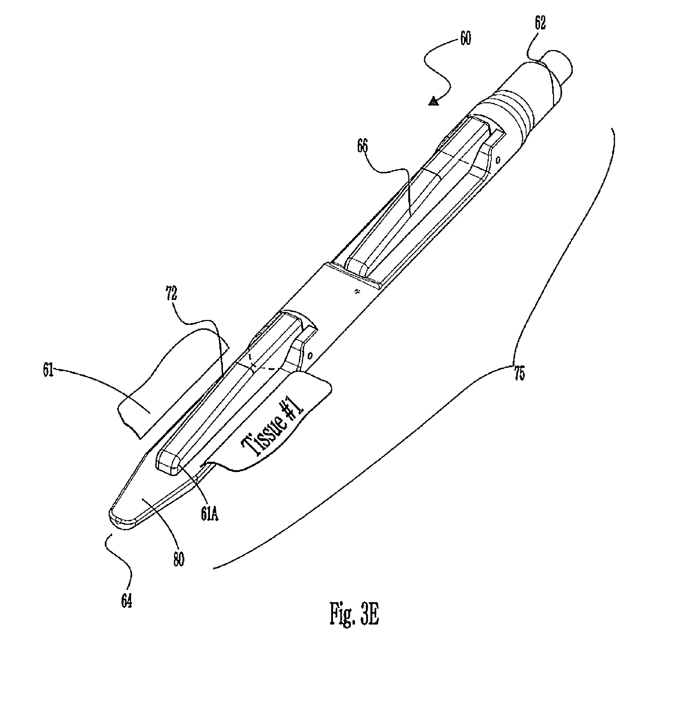

In FIG. 3B, the second closing member 72 is open, whereas the first closing member 66 is closed. End effector assembly 60 approaches a first tissue 61. In FIG. 3C, first tissue 61 is located in the vicinity of the second closing member 72. In FIG. 3D, a first tissue sample portion 61A of the first tissue 61 is disposed within the second cavity 74. In FIG. 3E, the second closing member 72 is closed such that the first tissue sample portion 61A is severed from the surrounding tissue by the blade on closing member 72 and is enclosed (sealed) and secured within the second cavity 74.

However, the surgeon may require another tissue sample from a different tissue during the same operation. As such, once the second closing member 72 has been closed to secure the first tissue sample 61, the first closing member 66 may be opened to allow the surgeon to obtain another tissue sample from the same or different tissue. For example, the actuating unit 21 of FIG. 1A may be employed to enable switching between the second and first closing members 72, 66. In particular, the knob 23 may slide from a first position to a second position via the sliding mechanism 25 in order to operatively connect handle 24 to the jaw closing rod for closing member 66 and disengage the closing rod for jaw 72, to operate the first closing member 66.

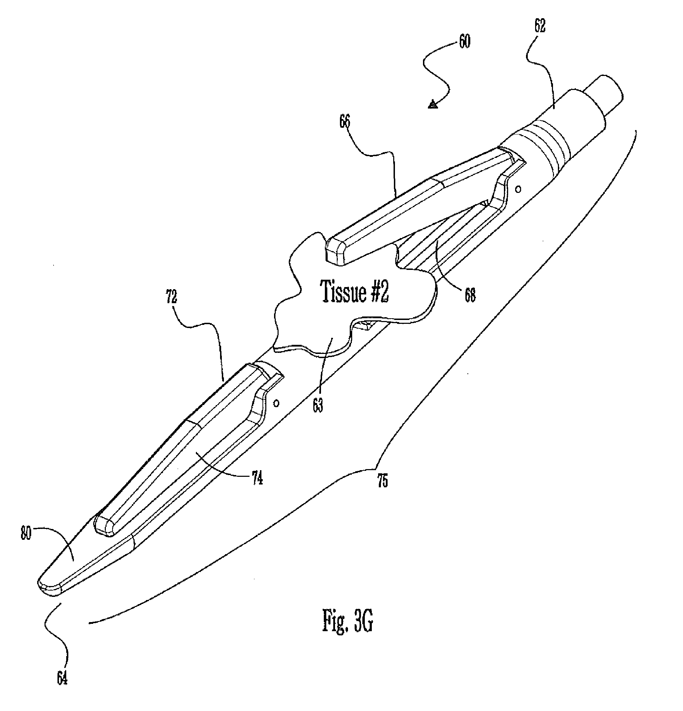

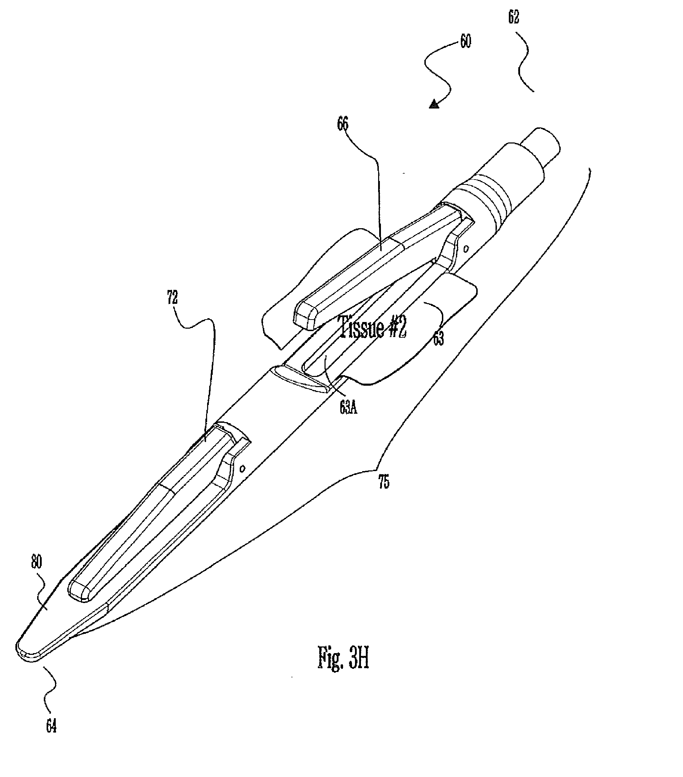

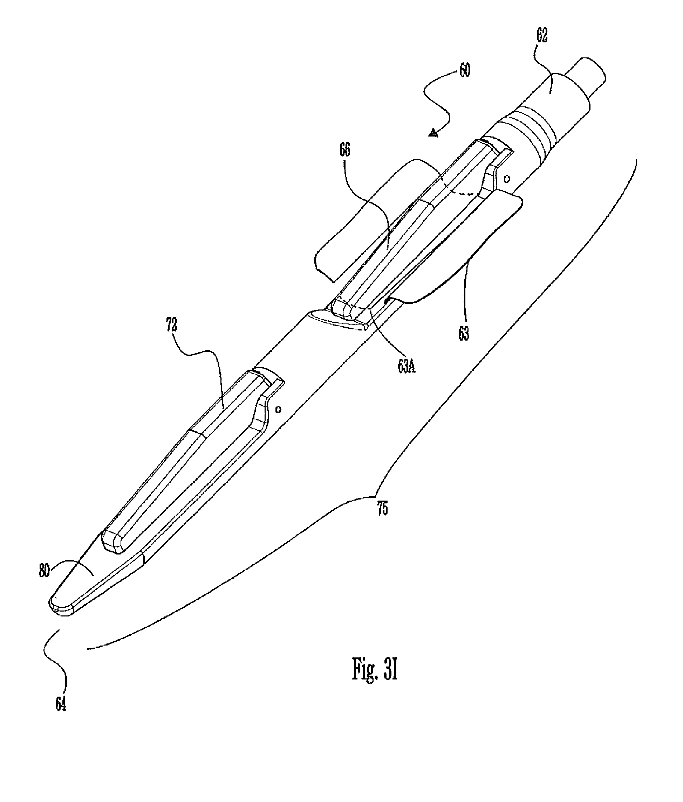

In FIG. 3F, the end effector assembly 60 approaches a second tissue 63. In FIG. 3G, first tissue 63 is located in the vicinity of the first closing member 66. In FIG. 3H, a second tissue sample portion 63A of the second tissue 63 is disposed within the first cavity 68. In FIG. 3I, the first closing member 66 closes such that the second tissue sample portion 63A is severed from the surrounding tissue and enclosed (sealed) and secured within the first cavity 68.

Therefore, the surgeon may obtain two tissue samples via the same end effector assembly during a single surgical operation by simply using an actuating unit to switch between first and second closing members. As an alternative to the switching mechanism, a separate handle can be provided to open and close each closing member.

Referring to FIGS. 4A and 4B, an end effector assembly 90 with opposed cavities 92, 102 positioned therein is illustrated, in accordance with yet another embodiment of the present disclosure.

The end effector assembly 90 includes a pair of opposed cavities 92, 102. The end effector assembly 90 also includes a first tip 100 and a second tip 101 at its distal end 107. Together, the tips 100, 101 form a blunt dissecting top similar to tip 50 of FIG. 1. The first cavity 92 is disposed on a top length of the end effector assembly 90, as viewed in the orientation of FIG. 4B, whereas the second cavity 102 is disposed on the opposing side, i.e. the bottom length, of the end effector assembly 90. The cavities 92, 102 are configured to receive different tissue samples therein. A first closing member 94 (or movable jaw) is pivotally mounted via a pivot mechanism 96, e.g. a pivot pin, to the elongate member or stationary jaw 103 of the end effector assembly 90. A second closing member 104 (or jaw) is pivotally mounted via a pivot mechanism 106, e.g. a pivot pin, to the elongate member 103 of the end effector assembly 90. The closing members 94, 104 each have a cutting blade (e.g. blade 105 of jaw 104) as in the cutting blade (and its variations) described above. The closing members 94, 104 are configured to securely enclose different tissue samples removed from tissue during a surgical procedure. The closing members 94, 104 are adapted and dimensioned to be of a size approximately equal to the size of their respective cavities 92, 102 to encapsulate and secure a tissue sample therein.

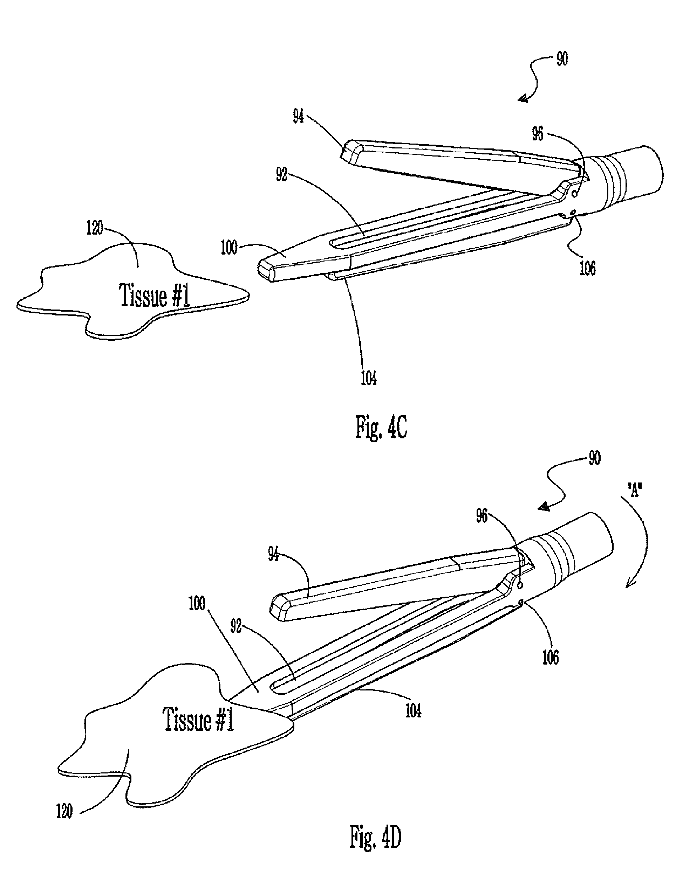

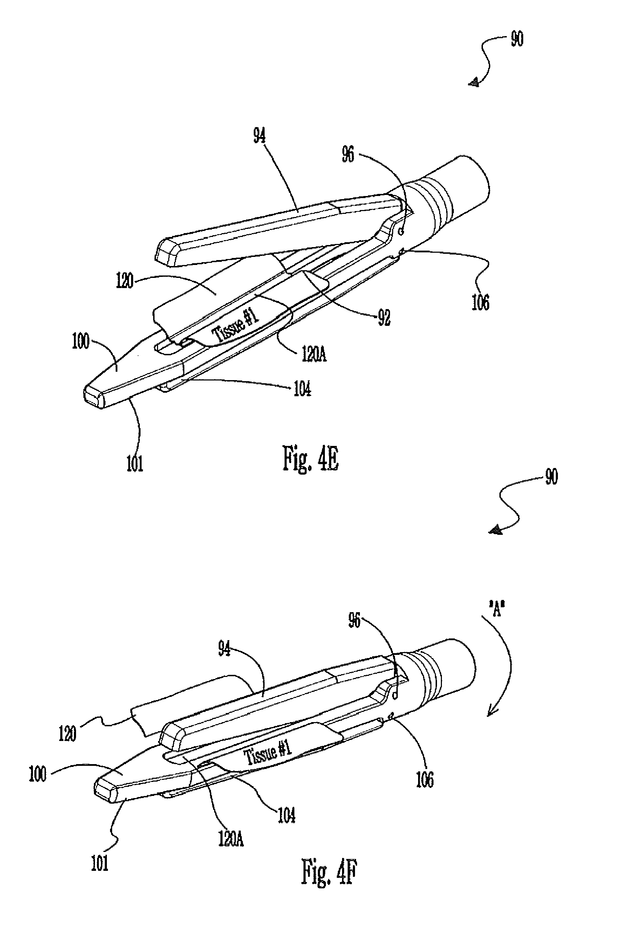

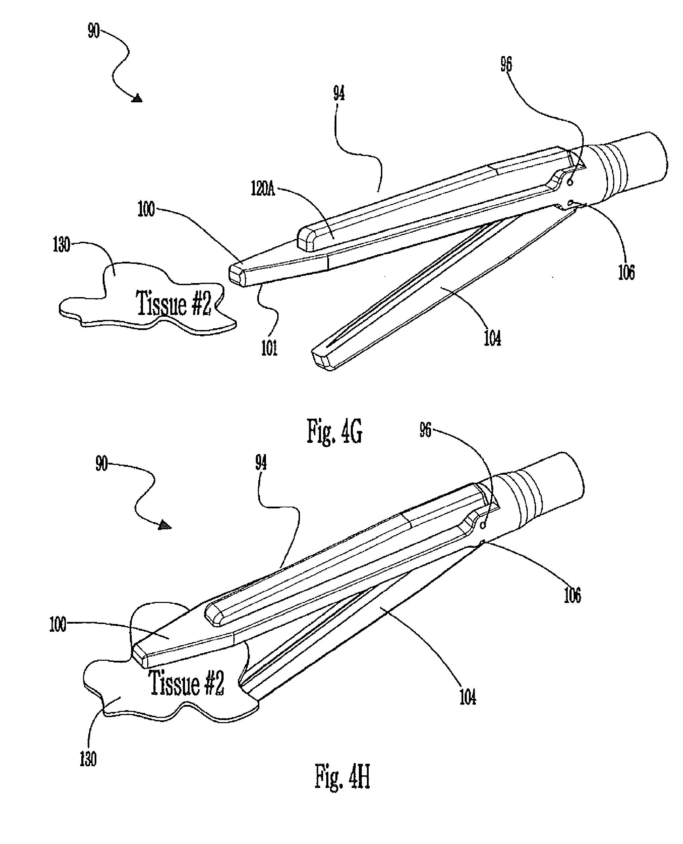

In use, the end effector 90 is at the distal end of the surgical instrument such as instrument 10 of FIG. 1. After end effector 90 of surgical instrument 10 is inserted in the thoracic cavity, e.g. through an access port, the surgeon advances end effector assembly 90 toward the target site (FIG. 4C). End effector assembly 90, extending from the distal end of elongate body 14 is then maneuvered within a working space to perform the desired diagnostic or surgical procedure, such as, removed of a lymph node from tissue. The end effector 90 can be rotated or articulated by knobs 28 and 13 respectively. For example, initially, both closing members 94, 104 may be in a closed configuration. Once the surgeon determines that a first sample needs to be extracted, first closing member 94 is adjusted to an open configuration for receiving the lymph node as shown in FIG. 4D. Once it is determined that the lymph node has been successfully placed within the first cavity 92, the handle member 24 is moved to cause the first closing member 94 to move to the closed configuration to sever the tissue sample (via blade 105) and retain it within cavity 92 (FIG 4E ad 4F). Then, if the surgeon determines that a second sample (e.g. tissue #2 of FIGS 4G and 4H) needs to be extracted, end effector 90 is rotated, as shown by arrow "A" in FIG. 4D. Then, the second closing member 104 is moved to an open configuration for receiving the lymph node from a different tissue. Once it is determined that the second lymph node has been successfully placed within the second cavity 102, the handle member 24 is moved to cause the closing member 104 to move to a closed configuration to sever the tissue sample and retain it within cavity 102. Once the lymph nodes have been secured within their respective cavities 92, 102, the end effector assembly 90 is removed from the patient. The lymph nodes are delivered to a technician for further processing to determine, for example, whether the lymph nodes are cancerous. Thus, the surgeon is permitted to extract two different tissue samples from different tissue during a single procedure without having to remove the end effector assembly each time a tissue sample needs to be extracted.

The design of the distal tips 100, 101 aids in the dissection of tissue. The tips 100, 101 can each be constructed in a sloping configuration. The tips 100, 101 may each have a conical or spherical outer face or edge. One skilled in the art may contemplate a plurality of different geometric configurations for the tips 100, 101 for enabling the tips 100, 101 to effectively dissect a portion of tissue or different tissues. The tip 100 may be disposed as a mirror image to tip 101, and adjacent to each other.

FIGS. 4C-4F illustrate the first cavity 92 of FIG. 4A receiving a first tissue sample portion 120A of tissue 120, whereas FIGS. 4G-4J illustrate the second cavity 102 of FIG. 4B receiving a second tissue sample portion 130A of tissue 130.

In FIG. 4C, the first closing member 94 is opened, whereas the second closing member 104 is closed. End effector assembly 90 approaches a first tissue 120. In FIG. 4D, first tissue 120 is located in the vicinity of the first closing member 94. In FIG. 4E, a first tissue sample portion 120A of the first tissue 120 is disposed within the first cavity 92. In FIG. 4F, the first closing member 94 closes such that the first tissue sample 120A is severed and enclosed and secured within the first cavity 92. Thus, a first tissue sample 120A has been obtained from the surgeon.

However, the surgeon may require another tissue sample during the same operation. As such, once the first closing member 94 has been closed to secure the first tissue sample 120, the end effector assembly 90 may be rotated, and the second closing member 104 may be opened to allow the surgeon to pursue another tissue sample from the same or different tissue. For example, the actuating unit 21 of FIG. 1A may be employed to enable switching between the first and second closing members 94, 104. In particular, the knob 23 may slide from a first position to a second position via the sliding mechanism 25 in order to rotate and access and operate the second closing member 104. Alternatively, a separate handle mechanism can be provided for each closing member.

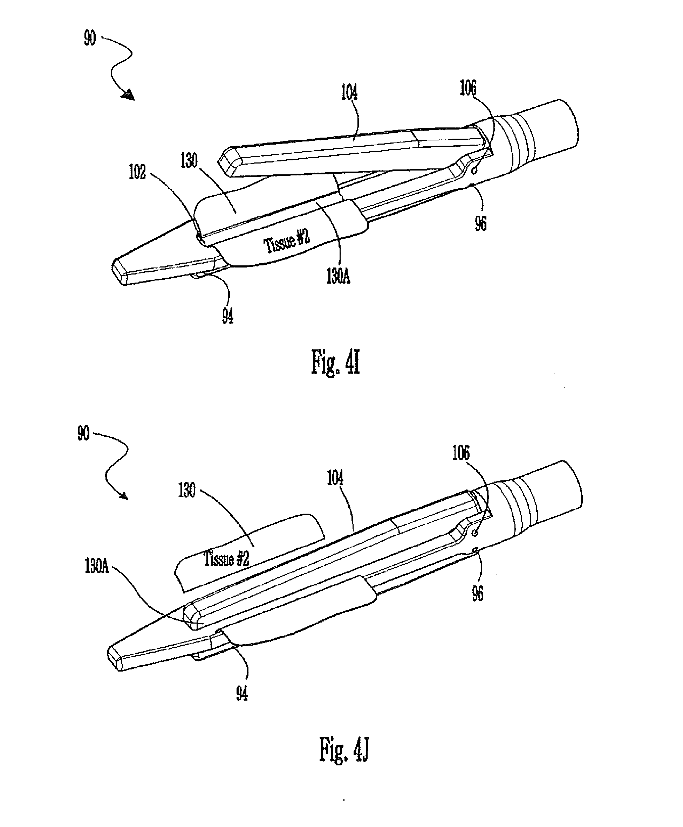

In FIG. 4G, the end effector assembly 90 approaches a second tissue 130. In FIG. 4H, second tissue 130 is located in the vicinity of the second closing member 104. In FIG. 4I, a second tissue sample portion 130A of the second tissue 130 is disposed within the second cavity 102. In FIG. 4J, the second closing member 104 closes such that the second tissue sample portion 130A is severed and enclosed and secured within the second cavity 102. Thus, a second tissue sample 130A has been obtained from the surgeon.

Therefore, the surgeon may obtain two tissue samples via the same end effector assembly during a single surgical operation by simply using an actuating unit to switch between first and second closing members. Note the second closing member can be operated either after or before operation of the first closing member.

Therefore, in summary, the exemplary embodiments of the present disclosure assist in the dissection and capturing for retrieval of lymph nodes during, for example, a VATS procedure. Lymph nodes are retrieved for specimen pathological analysis to help plan for further cancer treatments. The end effector assemblies of the surgical instrument 10 would be able to navigate through the thoracic cavity to dissect tissue and capture and remove lymph nodes. The surgical instrument 10 would be navigated by a surgeon with the jaws held in the closed position by the handle, such that the tip of the distal end of the end effector assembly dissects tissue and is advanced to be placed adjacent the lymph node. Once the handle of the surgical instrument 10 is released, the closing member is opened and the cavity is exposed to receive the tissue dissected. The handle of the surgical instrument 10 is then actuated to close the closing member and cut or separate the specimen (e.g., a lymph node) from the surrounding tissue. The surgical instrument 10 is then removed from the body of the patient and the specimen is removed from the cavity and placed in, for example, a proper receptacle for further examination. The distal end of the surgical instrument 10 is configured to articulate as well as rotate about a longitudinal axis of the elongate body 14 of the surgical instrument 10 to facilitate maneuvering the closing members to the desired position adjacent tissue to be removed.

It is also contemplated that in some embodiments, the jaws of the foregoing embodiments can be provided with electrocautery capability to cut tissue and/or effect a tissue seal.

As can thus be seen, the present disclosure provides a system for capturing one or more body tissue samples. In some embodiments, more than one body tissue sample may be captured during a single procedure, thus shortening the surgical procedure.

As can be appreciated, although described for thoracic procedures, the surgical instruments disclosed herein can be utilized in other procedures and other body cavities.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of presently disclosed embodiments. Thus the scope of the embodiments should be determined by the appended claims and their legal equivalents, rather than by the examples given.

Persons skilled in the art will understand that the devices and methods specifically described herein and illustrated in the accompanying drawings are non-limiting exemplary embodiments. The features illustrated or described in connection with one exemplary embodiment may be combined with the features of other embodiments. Such modifications and variations are intended to be included within the scope of the present disclosure. As well, one skilled in the art will appreciate further features and advantages of the present disclosure based on the above-described embodiments. Accordingly, the present disclosure is not to be limited by what has been particularly shown and described, except as indicated by the appended claims.

The invention may be described by reference to the following numbered paragraphs:-
1. A surgical instrument comprising:
   a handle portion;
   a body portion extending distally from the handle portion and defining a longitudinal axis; and
   an end effector assembly disposed at a distal end of the body portion, the end effector assembly including:
      a distal tip configured for dissection of tissue
      an elongate member having a cavity for the reception of a tissue sample therein;
   and
      a closing member for securely enclosing the tissue sample within the cavity.
2. The surgical instrument according to Paragraph 1, wherein the end effector assembly is rotatable about the longitudinal axis.
3. The surgical instrument according to Paragraph 1, further comprising an articulation mechanism for articulating the end effector assembly with respect to the longitudinal axis.
4. The surgical instrument according to Paragraph 1, wherein the closing member includes a blade member.
5. The surgical instrument according to Paragraph 1, wherein the closing member is configured to fit inside the cavity.
6. The surgical instrument according to Paragraph 1, wherein the closing member is pivotally coupled to the elongate member.
7. The surgical instrument according to Paragraph 1, wherein the elongate member includes a second cavity, each cavity dimensioned and adapted to receive a different tissue sample, and each cavity including a respective closing member, wherein access to each cavity is controlled by an actuating unit.
8. The surgical instrument according to Paragraph 1, wherein the elongate member includes a second cavity, the cavities disposed on opposed sides thereof, each cavity dimensioned and adapted to receive different tissue samples, and each cavity including a respective closing member, wherein access to each cavity is controlled by an actuating unit.
9. The surgical instrument according to Paragraph 1, wherein the end effector assembly is removably attachable to the body portion.
10. A method of removing tissue from a thoracic cavity, the method comprising:
   providing a surgical instrument including:
      a handle portion;
      an elongated body portion extending distally from the handle portion and defining a longitudinal axis and having a dimension for insertion through an access part; and
      an end effector assembly disposed at a distal end of the body portion, the end effector assembly having an elongate member having a first cavity for the reception of a tissue sample therein and a closing member for securely enclosing the tissue sample within the cavity;
   introducing the surgical instrument into the thoracic cavity of a patient;
   dissecting tissue via a distal end of the elongate member;
   advancing the end effector assembly toward a target tissue within the thoracic cavity;
   opening the closing member to receive the target tissue within the cavity;
   closing the closing member to sever a tissue sample from the target tissue and to seal the tissue sample within the first cavity of the elongate member; and
   removing the end effector assembly from the patient with the tissue sample secured within the cavity.
11. The method according to Paragraph 10, further comprising the step of rotating the end effector assembly about the longitudinal axis.
12. The method according to Paragraph 10, further comprising the step of articulating the end effector assembly with respect to a longitudinal axis of the body portion.
13. The method according to Paragraph 10, wherein the end effector is removably coupled to the body portion.
14. The method according to Paragraph 10, wherein the instrument includes a second cavity formed on the elongate member, each cavity dimensioned and adapted to receive a different tissue sample, and the second cavity including a second closing member.
15. The method according to Paragraph 14, wherein the first and second cavities are disposed on opposed sides thereof, and the method includes the step of closing a second closing member to sever a second tissue sample from surrounding tissue and seal the severed second tissue sample in the second cavity.
16. The method according to Paragraph 14, wherein the first and second cavities are disposed in substantial longitudinal alignment, and the method includes the step of closing a second closing member to sever a second tissue sample from surrounding tissue and seal the severed second tissue sample in the second cavity.
17. The method according to Paragraph 10, wherein the surgical instrument is introduced into the thoracic cavity during a video assisted thoracic surgery procedure.
18. The method according to Paragraph 17, wherein the surgical instrument retrieves lymph node for pathology.

## Claims

1. A surgical instrument comprising:
a handle portion;
a body portion extending distally from the handle portion and defining a longitudinal axis; and
an end effector assembly disposed at a distal end of the body portion, the end effector assembly including:
a distal tip configured for dissection of tissue
an elongate member having a cavity for the reception of a tissue sample therein; and
a closing member for securely enclosing the tissue sample within the cavity.

2. The surgical instrument according to Claim 1, wherein the end effector assembly is rotatable about the longitudinal axis.

3. The surgical instrument according to Claim 1 or Claim 2, further comprising an articulation mechanism for articulating the end effector assembly with respect to the longitudinal axis.

4. The surgical instrument according to any preceding Claim, wherein the closing member includes a blade member.

5. The surgical instrument according to any preceding Claim, wherein the closing member is configured to fit inside the cavity.

6. The surgical instrument according to any preceding Claim, wherein the closing member is pivotally coupled to the elongate member.

7. The surgical instrument according to any preceding Claim, wherein the elongate member includes a second cavity, each cavity dimensioned and adapted to receive a different tissue sample, and each cavity including a respective closing member, wherein access to each cavity is controlled by an actuating unit.

8. The surgical instrument according to Claim 7, wherein the elongate member includes a second cavity, the cavities disposed on opposed sides thereof, each cavity dimensioned and adapted to receive different tissue samples, and each cavity including a respective closing member, wherein access to each cavity is controlled by an actuating unit.

9. The surgical instrument according to any preceding Claim, wherein the end effector assembly is removably attachable to the body portion.
